# EUROPEAN PATENT SPECIFICATION

(11) **EP 1 682 069 B1**
(45) Date of publication and mention of the grant of the patent: **08.07.2009**
(21) Application number: 04762943.1
(22) Date of filing: 21.10.2004
(51) Int. Cl.: A61J 1/05, A61M 5/142

(54) **RESERVOIR DEVICE WITH INCLINED NEEDLE**
RESERVOIRVORRICHTUNG MIT GENEIGTER NADEL
DISPOSITIF A RESERVOIR AVEC AIGUILLE INCLINEE

(30) Priority: 21.10.2003 DK 200301546; 10.11.2003 US 518837 P
(43) Date of publication of application: 26.07.2006
(73) Proprietor: NOVO NORDISK A/S, 2880 Bagsvaerd (DK)
(72) Inventor: SPARHOLT, Philip, Albert, DK-2765 Sm rum (DK); ETHELFELD, Erik, Winkel, DK-1360 Copenhagen K (DK)
(74) Representative: Kristoffersen, Bo
(86) International application number: PCT/DK2004/000724
(87) International publication number: WO 2005/037185

(56) References cited:
- CH-A- 337 990
- FR-A- 1 144 814
- FR-A- 2 752 410
- US-A- 3 642 047
- US-A- 4 886 499
- US-A- 6 074 369

## Description

The present invention generally relates to an apparatus comprising a reservoir for the storage of fluids, the reservoir comprising an outlet allowing the interior of the reservoir to be accessed by a fluid conducting structure such as a penetrating needle member. The reservoir may be designed to contain in particular medical liquid products such as drugs, drug solutions, infusion solutions, parenteral solutions, dialysis solutions, perfusion solutions, chemical and alimentary liquids, human blood and its fractions, and the like. The reservoir may also be used for other purposes, e.g. calibration liquids for analytical equipment.

### BACKGROUND OF THE INVENTION

In the disclosure of the present invention reference is mostly made to liquids in the form of medical liquids as outlined above, however, this is only an exemplary use of the present invention.

A medical liquid is often supplied in a reservoir (e.g. a container, bag or bottle) which can be accessed by means of a hollow needle, the needle typically penetrating a dedicated connection element (or access means) of the reservoir to provide a fluid communication with the interior of the reservoir. The needle access may be used either for withdrawing liquids from the reservoir or for supplying a liquid to the reservoir. For example, when preparing the fluids which are to be administrated to the body of a patient from a given reservoir, it is common that medically effective substances are supplied to a pre-sealed reservoir which is filled with a transport fluid, usually in the form of sodium chloride solution or a glucose solution, the diluted drug then being given to a patient intravenously via an intravenous (IV) infusion set. For this type of use, the reservoir may be provided with a single connection means adapted to be used for both purposes, or the reservoir may comprise two connection means adapted for their respective purposes, e.g. for a larger infusion set outlet needle and a smaller drug injecting needle. The connection element may be adapted to be opened manually to provide an opening, through which a needle subsequently can be inserted, or the pointed needle can be used for penetrating the connection element, which may be of the self-sealing type, e.g. the connection element will seal the reservoir after the needle has been withdrawn.

In case the reservoir is of glass, the connection element will be a separate element which is mounted to the glass reservoir by special means, however, for plastic reservoirs the connection element will typically be formed integrally with the reservoir. One of the most widely used type of plastic reservoirs for medical use is in the form of a flexible infusion bag comprising at a lower portion thereof one or more needle-penetratable connection elements. Such bags are typically formed from flexible foil sheets which are joined to form an internal space. Depending on the actual construction of the bag, the connection element(s) may be arranged either on a surface portion of a foil sheet or may be arranged corresponding to an edge portion of the reservoir. For the latter type, the connection element is typically positioned and held in place between two foil sheets connected to each other by welding.

For both of the above two arrangements, the self-sealing element is normally carried by a tubular member connected to the bag in either of the above two ways. For example, US patent 4,362,158 shows an infusion bag in which a tubular nozzle member is connected to an infusion bag corresponding to a free surface thereof, a self-sealing rubber seal member being mounted on the nozzle. CH 337 990 upon which the two-part form of claim 1 is based discloses a flexible reservoir in which a needle is inserted through a surface of the reservoir.

For some liquids, e.g. certain types of drugs, it is desirable if the elastomeric material from which the seal member normally is manufactured, does not come in contact with drug. To solve this problem, European patent application EP 0 364 783 describes a medicament bottle having an external sealing element held in place by a separate cap member attached to the outer surface of the bottle.

The above reservoirs are relatively large, typically comprising 100 - 1.000 ml of liquid, however, reservoirs which can be accessed by a penetrating needle member is also used for much smaller volumes. For example, certain calibration solutions for calibrating analytical equipment are supplied in small bag-like reservoirs containing a few ml. When properly designed, such small reservoirs may also be used for drug purposes.

### DISCLOSURE OF THE INVENTION

Having regard to the above-described reservoirs and devices, it is an object of the present invention to provide an apparatus comprising a reservoir with an outlet which can be used in an efficient manner. It is a further object of the invention to provide a reservoir which is convenient and safe in use and allows a varity of applications. It is a yet further object of the invention to provide a reservoir which can be used with a varity of liquids. Further objects and advantages will become apparent from the disclosure of the invention and the description of the exemplary embodiments.

In the disclosure of the present invention, embodiments and aspects will be described which will address one or more of the above objects or which will address objects apparent from the below disclosure as well as from the description of exemplary embodiments.

More specifically, the present invention provides in a first aspect an apparatus as defined in claim 1.

By the term "adapted to contain" is defined that a reservoir in accordance with the invention may be provided in an empty state for subsequent filling, or that it may be filled during manufacture of the reservoir.

The reservoir may be made from e.g. two separate foil members welded together at the periphery thereof (e.g. at four sides for a square reservoir), by a folded-over foil welded together at the remaining peripheral portion (e.g. at three sides for a square reservoir), or from a foil tube welded together at the two ends thereof. Thus, for a typical bag- or pouch-formed reservoir the welds (e.g. two opposed welds) can be used to define the general plane of the reservoir, although it would be possible to arrange the welds at other locations. When the reservoir is filled it will typically bulge out to provide one or more convex surfaces, which apart from the "top" area will be arranged inclined relative to the general plane. To present a portion of the foil surface which is more inclined than that of the reservoir in its "inherent" configuration, a portion of the reservoir is deflected relative to the remaining portion of the reservoir, e.g. bend along a line, this aiding the insertion of the needle into the reservoir.

The outlet may be provided by any suitable structure allowing a needle to be brought in fluid communication with the interior of the reservoir without. If the foil material from which the reservoir is formed would allow a needle to puncture the foil in a non-leaking manner no additional member would have to be provided, however, in most circumstances an additional outlet member will have to be provided.

For example, the reservoir outlet means may be in the form of a septum member formed from a needle-penetratable self-sealing material as described above, the septum advantageously being connected by welding. The septum may cover portions of the reservoir in addition to those forming the outlet, e.g. the septum may be arranged on a rounded edge portion of the reservoir thereby also covering a portion of the reservoir which is oriented perpendicularly relative to the general plane. Dependent upon the fluid to be contained in the reservoir, the walls thereof may comprise one or more layers as described above.

Although the term "self-sealing" will be clear to the skilled person, it should be noted that this is not to be regarded as an absolute term for a given septum but that it will depend upon the intended use for a given reservoir. For example, a given septum will be designed to be self-sealing in connection with needles of a given range of gauges (i.e. diameters) and with a given design for the pointed distal end. Thus, a relatively thin septum adapted to be used with a correspondingly thin needle may not be self-sealing when penetrated by a larger needle. Further, if the reservoir is pressurized above the intended internal pressure, a punctuated septum may leak.

In preferred embodiments the inclined angle is less than 45 degrees, preferably less than 30 degrees and more preferably less than 15 degrees (with parallel being defined as zero degrees). By introducing the needle at an inclined angle, the reservoir may collapse fully or partially without the needle penetrating the reservoir portion arranged opposite the outlet means.

If the fluid (liquid or gas) to be contained in the reservoir does not require specific properties for the surrounding walls (e.g. in respect of evaporation, leakage or chemical inertness), the first foil portion may be made from a single layer of material or the reservoir in general may be formed from a single material. If the fluid requires specific properties for the reservoir, the reservoir may be provided with an outer surface generally formed from a first material and an inner surface generally formed from a second material. The wall portion providing or comprising the inlet may be of a special construction (e.g. allowing needle-penetration) with the rest of the reservoir wall having different properties.

The foil members may be composite laminates of continuous layers including an outer layer and an inner layer. This would allow the outer surface to be optimized for connection of a septum member whereas the inner surface may be optimized in respect of reservoir properties in respect of the fluid to be contained. Any laminate referred to in the present application may be a traditional laminate, a co-extrudate or an extrusion-laminate. The walls defining the reservoir may be formed from a single material or single type of laminate, or different materials or laminates may be used for different wall portions of the reservoir.

The foil members may comprise an intermediate layer, the inner layer being formed from a weldable material, which would allow the foil members to be sealed together at least partially by welding corresponding to the peripheries of the bag. In case the outer layer solely provides the mounting surface for the septum, the "intermediate" layer may provide the outer layer for a portion of the reservoir. To allow a contained liquid to be viewed through the reservoir wall, at least a portion thereof may be formed by a transparent or translucent material.

Just as a portion of the outer reservoir wall surface may be optimized for attachment of the septum by welding, also the septum mounting surface may be optimized. For example, the septum may be a laminate comprising two or more layers, the "lower" layer providing the mounting surface. The septum may be welded to the reservoir corresponding to substantially the entire mounting surface, however, it may also be welded using one or more (concentric) circumferential welds.

The septum may be configured to provide additional properties in addition to the sealing properties. For example, a portion of the septum or the entire septum may be configured to flex together with the portion of the reservoir to which it is mounted, this allowing the septum to be bend either during manufacture or during use. To allow this flexibility the septum may be in the form of a relatively thin member or may comprise a relatively thin portion. Further, when the septum is arranged "naked" on the reservoir, it may be accessed for other purposes than providing a fluid connection as will be described below.

In an exemplary embodiment, the apparatus further comprises an expelling assembly adapted to expel a fluid (e.g. a drug) contained in the reservoir through the outlet of the reservoir. The expelling assembly may be adapted to force or suck the drug from the reservoir. In the latter case the expelling assembly may be in the form of a suction pump having an inlet and an outlet and an internal flow path arranged therebetween, the inlet being adapted to be arranged in fluid communication with the reservoir through the needle.

The reservoir and the expelling assembly may be arranged moveable relative to each other, or they may both be fixed relative to each other, the fluid communication being provided by a moveable fluid connector which advantageously is formed as part of the expelling assembly and serving as an inlet therefore. More specifically, a fluid connector may be arranged within the interior of the expelling assembly in an initial state, the fluid connector comprising an inlet and an outlet, wherein the fluid connector is arranged to be operated from the initial state and to an operating state in which fluid communication is established between the interior of the reservoir and the interior of the expelling assembly via the fluid connector and with the outlet of the fluid connector being arranged in the flow path of the expelling assembly.

The apparatus may be in the form of skin-mountable pump device further comprising a transcutaneous device adapted to penetrate the skin of a subject, a mounting surface adapted for application to the skin of the subject, wherein the reservoir comprises a fluid drug, and the expelling assembly, in a situation of use, is adapted for expelling the drug out of the reservoir and through the skin of the subject via the transcutaneous device. The transcutaneous device may be in the form of a pointed hollow infusion needle, a micro needle array, a pointed needle sensor, or a combination of a relatively flexible *per se* blunt cannula or sensor device with a pointed insertion needle may provide a pointed transcutaneous device, the insertion needle being retractable after insertion of the blunt portion of the transcutaneous device. The cannula is advantageously soft and flexible relative to the insertion needle which typically is a solid steel needle. In the disclosure of the present invention as well as in the description of the exemplary embodiments, reference will mostly be made to a transcutaneous device in the form of an infusion needle. The reservoir may be supplied pre-filled to the user or adapted to be filled (and refilled) by the user.

The present disclosure also describes a method of connecting a flexible reservoir with a needle, comprising the steps of providing a flexible reservoir having a pouch-like configuration defining a general plane, the reservoir comprising a needle-penetratable outlet arranged on a portion of the reservoir inclined relative to the general plane, providing a needle having a generally straight inlet portion, and inserting the needle through the inlet substantially in parallel with the general plane thereby establishing .a fluid communication between the needle and the reservoir.

As discussed above, a "naked" septum arrangement may be adapted to provide additional functionality to a reservoir. Correspondingly, in a further aspect of the invention an apparatus is provided comprising a housing, a flexible reservoir adapted to contain a fluid and comprising a septum member formed from a needle-penetratable self-sealing material, at least a portion of the flexible reservoir being arranged within the housing, as well as mounting means arranged within or formed by the housing, wherein the mounting means engages the septum member to thereby mount the flexible reservoir relative to the mounting means. In this way a secure fixation between the reservoir and the mounting means is provided without having to interfere with the general flexibility of the reservoir, e.g. substantially the entire flexible reservoir apart from the outlet/mounting means may be arranged free to move relative to the housing, this allowing the reservoir to be emptied to a high degree. Typically, the housing will be closed with the reservoir arranged there within, however, in principle it may be open in which case the housing would serve as a supporting structure which in the context of the present invention is included in the term housing.

In exemplary embodiments the above-described mounting arrangement may be used in combination with an expelling assembly and a transcutaneous device to provide a delivery device as described above.

As described above, a reservoir comprising a septum is provided in combination with additional structures for mounting and/or connecting the reservoir. Thus, corresponding to a further aspect of the disclosure, a reservoir *per se* is described adapted to contain a fluid within a cavity formed by walls, comprising a first wall portion formed from a needle-penetratable material, where the wall portion comprises an outer first mounting surface. The reservoir further comprises a septum member (in the following also referred to as septum) formed from a needle-penetratable self-sealing material, the septum member comprising a second mounting surface, wherein the septum member, corresponding to the second mounting surface, has been mounted on the first mounting surface by means of welding together the two surfaces. The material for the first mounting surface may be located solely corresponding to the placement of the septum, or it may cover a portion or the entire exterior surface of the reservoir. The reservoir may be a relative rigid reservoir (e.g. a blown bottle) or a more flexible reservoir as a traditional IV bag or a reservoir as described above.

Although the septum may be mounted alone, it may be desirable to provide additional mounting means allowing access means such a tubing to be connected to the reservoir, e.g. as described above with respect to the known IV bags. For example, a tubular connector protruding from the surface of the reservoir may be attached around the septum, e.g. circumferentially. In this way the two components can be attached independently of each other without having the septum to be mounted to the connector, although it may be convenient to attach the two components to the reservoir simultaneously during a welding procedure.

The septum may also be configured to serve additional purposes. For example, in case the reservoir is in the form of a flexible reservoir adapted to be mounted in a drug delivery device as described above, the septum may be used to handle the reservoir during the mounting procedures just as the septum may be adapted to serve as a connecting means between the reservoir and the delivery device, e.g. the septum member may be welded to the delivery device or the septum member and the delivery device may be provided with mating coupling means.

The septum and the materials forming the reservoirs in accordance with the different aspects of the present invention may be selected in accordance with the above described embodiments and their intended use. For example, the wall portion providing the first mounting surface may be manufactured from or comprise polyethylene (PE), polypropylene (PP), oriented polypropylene (OPP), amorphous polyester (PET), oriented amorphous polyester (OPET), polyamide (PA) or oriented polyamide (OPA). The septum portion forming the second mounting surface may be manufactured from a thermoplastic elastomeric (TPE) material, e.g. an oleophenic based thermoplastic material or an oleophenic based thermoplastic vulcanisate, or blends thereof. More specifically, the TPE may be a thermoplastic vulcanisate composed of bromo-butyle rubber in a polypropylene matrix (e.g. as sold under the trade name Trefsin®), a thermoplastic vulcanisate composed of EPDM rubber in a polypropylene matrix (e.g. as sold under the trade name Santoprene®), or a styrene ethylene butylene styrene (SEBS) copolymer based elastomere (e.g. as sold under the trade name Kraiburg®). Also TPU, TPE-A or TPE-E may be used.

As used herein, the term "drug" is meant to encompass any drug-containing flowable medicine capable of being passed through a delivery means such as a hollow needle in a controlled manner, such as a liquid, solution, gel or fine suspension. Representative drugs include pharmaceuticals such as peptides, proteins, and hormones, biologically derived or active agents, hormonal and gene based agents, nutritional formulas and other substances in both solid (dispensed) or liquid form. In the description of the exemplary embodiments reference will be made to the use of insulin. Correspondingly, the term "subcutaneous" infusion is meant to encompass any method of transcutaneous delivery to a subject.

Herein the term "insulin" refers to insulin from any species such as porcine insulin, bovine insulin, and human insulin and salts thereof such as zinc salts, and protamin salts as well as active derivatives of insulin, and insulin analogues. The term "active derivatives of insulin", is what a skilled art worker generally considers derivatives, vide general textbooks, for example, insulin having a substituent not present in the parent insulin molecule. The term "insulin analogues" refers to insulin wherein one or more of the amino acid residues have been exchanged with another amino acid residue and/or from which one or more amino acid residue has been deleted and/or from which one or more amino acid residue has been added with the proviso that said insulin analogue has a sufficient insulin activity.

The material(s) used to form the reservoir or reservoir devices of the present invention may be selected in accordance with the intended use. Thus it may be required that the material(s) fulfil(s) specified functional requirements such as physical properties for the material after sterilization, chemical requirements for the material after sterilization, and cleanliness. Correspondingly, the material may be sterilizeable using e.g. gamma irradiation, electron beam, steam, or ethylene oxide. The material(s) may further be selected in accordance with one or more of the following requirements: 1) the material must be transparent, 2) the material must provide a good barrier against water evaporation; 3) the material must provide a good barrier against gasses (for example, oxygen and carbon dioxide); 4) the material must provide a good barrier against preservatives (for example, phenol and meta-cresol); 5) the material must provide a good barrier against odors (for example preservatives); 6) the material must be resistant against environmental stress cracking (for example, oils, perfumes); 7) the material must be resistant against flex-crack; 8) the material must have good sealing properties (for example, by welding); 9) the material must not delaminate after sterilization, during processing or storage;10) the material must not relax significantly during storage and use, 11) the material must not emit substances to the drug which can affect the health and safety of the patient (leachables); 12) the material must have a very low level of extractables; and 13) the material must be compatible with a contained drug formulation. It may further be relevant that the material(s) fulfil(s) certain health and safety requirements, preferably most of or all the requirements mentioned in 1) European Pharmacopoeia (Ph. Eur.) 2002, 4th edition; 2) The United States Pharmacopeia (USP) 25; 3) Japanese Pharmacopeia (JP) XIV; 4) EEC Directive 90/128 + amendments "Relating to plastics materials and articles intended to come into contact with foodstuffs"; 5) Code of federal regulations (CFR) Title 21 Food and Drugs, part 170 - 190; 6) III/9090/90 EN. Plastic Primary Packaging Materials. Note for Guidance; and 7) Guidance for Industry. Container Closure Systems for Packaging Human Drugs and Biologics, Chemistry, Manufacturing, and Controls Documentation. FDA, May 1999.

In respect of laminates the following definitions are used: Co-extrusion covers a process where two or more polymer materials are melded in two or more extruders and co-extruded together through a flat nozzle or systems of flat nozzles and cooled to form the co-extruded foil. Extrusion-lamination covers a process where a feedstock in form of a foil of one material is coated through a flat nozzle or systems of flat nozzles from one or more extruders with one layer or more layers of melted material or materials and then cooled to form the extrusion-lamination foil. "Traditional" lamination covers a process, where two feed stocks of foil materials are joined together by adding a proper adhesive to one foil, followed by addition of the second foil forming the laminated foil. A tie layer is a layer which is placed between two polymer layers with the object of securing that the two layers are joined together.

### BRIEF DESCRIPTION OF THE DRAWINGS

In the following exemplary embodiments will be further described with references to the drawings, wherein
fig. 1 shows a perspective view of a drug delivery device,
fig. 2 shows a perspective view of a further drug delivery device,
figs. 3 and 4 show a pump unit and reservoir connection,
fig. 5A shows a schematic overview of a pump connected to a reservoir,
fig. 5B shows an exploded view of a pump assembly,
fig. 5C shows a cross-sectional view of the pump assembly of fig. 5C,
figs. 5D and 5E show partial cross-sectional views of the pump assembly of fig. 5C,
fig. 6A shows a perspective view of a flexible reservoir,
fig. 6B shows a side view of the reservoir shown in fig. 6A,
fig. 6C shows an upper view of the reservoir shown in fig. 6A,
fig. 7A shows a side view of a reservoir with a needle inserted,
fig. 7B shows an upper perspective view of the reservoir of fig. 7A,
fig. 7C shows a lower perspective view of the reservoir of fig. 7A, and
fig. 8 shows an exploded view of a further reservoir unit.

In the figures like structures are mainly identified by like reference numerals.

### DESCRIPTION OF EXEMPLARY EMBODIMENTS

When in the following terms as "upper" and "lower", "right" and "left", "horizontal" and "vertical" or similar relative expressions are used, these only refer to the appended figures and not to an actual situation of use. The shown figures are schematic representations for which reason the configuration of the different structures as well as the relative dimensions are intended to serve illustrative purposes only.

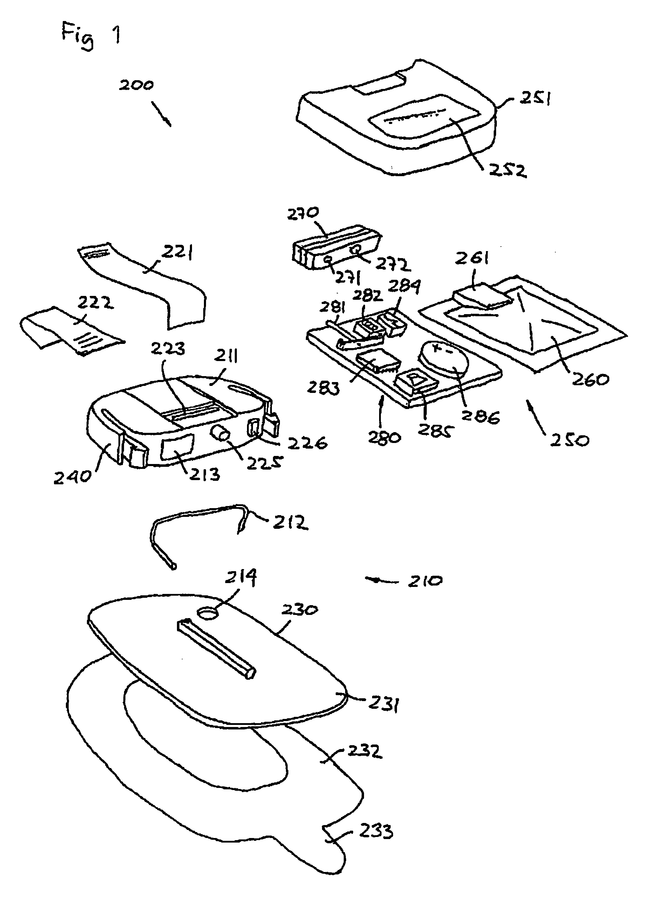

In fig. 1 an embodiment of a drug delivery device is shown, the device comprising a flexible reservoir allowing one or more aspects of the present invention to be implemented.

More specifically, fig. 1 shows in an exploded perspective view a medical device in the form of a drug delivery device 200 comprising a needle unit 210 having a needle housing 211, a base member 230 with a lower mounting surface adapted for application to the skin of a subject, and a separate reservoir and pump unit 250. In the shown embodiment the base member comprises a relatively rigid upper portion 231 attached to a more flexible adhesive patch member 232 provided with a gripable strip and having a lower adhesive surface providing the mounting surface *per se*. In the shown embodiment the needle housing is attached to the base plate as a separate unit, the two elements in combination forming the needle unit. Within the housing a hollow infusion needle 212 is pivotally arranged.

The needle unit comprises first and second openings 213, 214 which may be open or covered by needle penetratable membranes to provide a sealed interior. The needle comprises a proximal inlet end and a pointed distal outlet end. The housing further comprises actuation means (not shown) for moving the needle between a retracted and extended state, and retraction means (not shown) for moving the needle between the extended and a retracted position. The actuation and retraction means are actuated by gripable first and second strip members 221, 222 connected to the respective means through slot-formed openings in the housing, of which the slot 223 for the first strip can be seen. The second strip is further connected to the patch strip 233. Arranged on the housing is user-actuatable male coupling means 240 in the form of a pair of resiliently arranged hook members adapted to cooperate with corresponding female coupling means on the reservoir unit. The housing further comprises connecting means 225 for establishing fluid communication between the pump unit and the reservoir (see below), and communication means 226 for activating and deactivating the expelling means.

The reservoir unit 250 comprises a housing 251 in which a reservoir and expelling means are arranged, the expelling means comprising a pump unit 270 and control and actuation means 280 therefore. The reservoir 260 is in the form of prefilled, flexible and collapsible pouch comprising a needle-penetratable septum 261 welded thereto and adapted to be arranged in fluid communication with the pump unit via a pump inlet 272. The reservoir is in the form of a flat pouch arranged substantially in parallel with the general plane of the device, the septum being mounted on an initially convex upper surface of the reservoir, thereby allowing a needle to be introduced therethrough substantially in parallel with the upper and lower walls of the reservoir. The housing comprises mounting means (not shown) allowing the septum to be fixated relative thereto, and a window 252 allowing the user to inspect the content of the reservoir. The shown pump is a mechanically actuated membrane pump, however, the expelling means may be of any suitable configuration.

The control and actuation means, which may be arranged on a PCB or flex-print, comprises a pump actuating member 281 in the form of a lever and piston arrangement driven by a coil actuator 282, a microprocessor 283 for controlling, among other, the pump actuation, a contact switch 284 cooperating with the communication means 226 on the needle unit, signal generating means 285 for generating an audible and/or tactile signal, and an energy source 286.

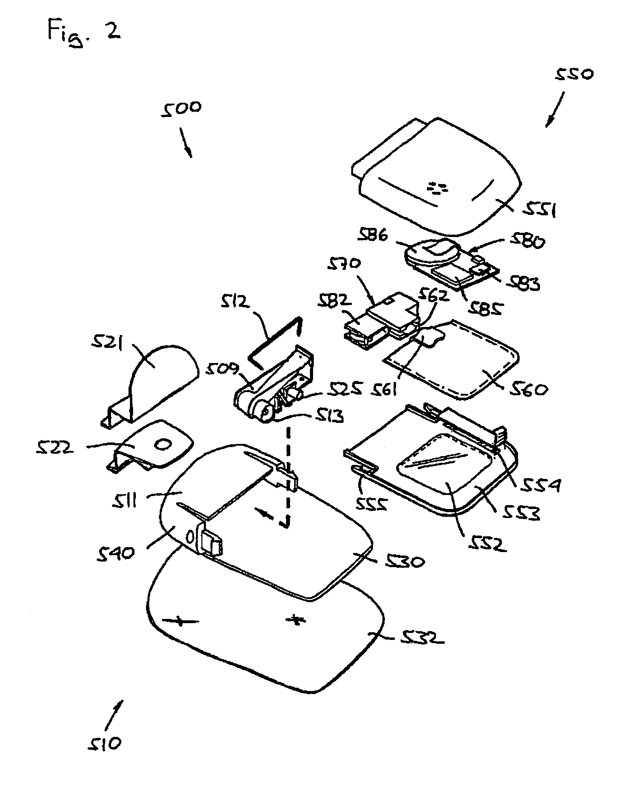

In fig. 2 a drug delivery device having the same general configuration as in fig. 1 is shown. More specifically, fig. 2 shows a drug delivery device 500 comprising a needle unit 510 having a housing portion 511 and a relatively rigid base portion 530 attached to a more flexible adhesive patch member 532 having a lower adhesive surface providing the mounting surface *per se*. Within the housing a needle actuation unit 509 is arranged, the needle actuation unit comprising a hollow infusion needle 512 pivotally arranged relatively to the base plate.

The inlet portion of the needle is arranged corresponding to the pivoting axis, the inlet portion being protected by a cylindrical member 513 protecting the user against accidental needle pricks. Adapted to cooperate with the actuation and retraction strips 521, 522 the needle actuation unit 509 comprises actuation means for moving the needle between a retracted and extended state, and retraction means for moving the needle between the extended and a retracted position. The needle unit further comprises male 540 and female (not shown) coupling means adapted to cooperate with corresponding female and male 555 coupling means on the reservoir unit, as well as connecting means 525 for establishing fluid communication between the pump unit and the reservoir (see below).

The reservoir unit 550 comprises a housing, formed from upper and lower housing portions 551, 553, in which a reservoir and expelling means are arranged, the expelling means comprising a pump unit 570 and control means 580 therefore. The lower housing portion comprises two windows 552, 554 allowing the user to inspect the content of the reservoir respectively a reservoir indicator (see below). The reservoir 560 is in the form of prefilled, flexible and collapsible pouch formed from a flexible foil folded corresponding to one edge of the reservoir and sealed along the remaining three edges, the reservoir comprising a needle-penetratable septum 561 welded thereto corresponding to the rounded, folded edge. The pump unit which in the shown embodiment is in the form of a membrane pump comprises a pump actuating member in the form of a coil actuator 582 operatively connected to thereto, and a mounting means in the form of a slot 562 allowing the septum to be mounted and fixed relative to the inlet means of the pump. The pump unit comprises a moveable fluid connector (see below) arranged to penetrate an inclined portion of the septum 561.

The control means comprises a microprocessor 583 for controlling, among other, the pump actuation, a signal generating means 585 for generating an audible and/or tactile signal, and an energy source 586. A reservoir indicator 582 for indicating to the user an amount of drug left in the reservoir is coupled to the control means. The indicator may be in the form of an electrochemical strip of the type used in e.g. batteries.

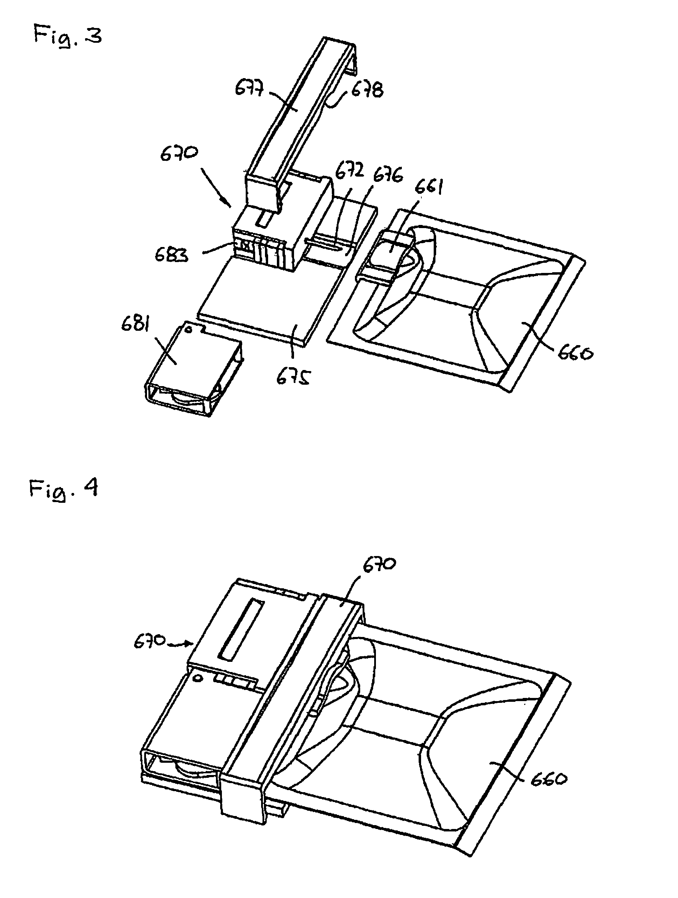

With reference to figs. 3 and 4 an alternative configuration for the reservoir mounting means is shown. The pump unit 670 comprises a base plate 675 with a first groove portion 676 and a clamp member 677 with a second groove portion 678, the two groove portions being adapted to engage opposed surfaces of the septum member 661 of a reservoir 660 when the clamp member is locked to the base plate as shown in fig. 4, thereby placing a securing the septum member relative to a fluid connector 672 forming the pump inlet. As appears, a portion of the septum is arranged on an inclined portion of the reservoir. The groove portions may be provided with additional gripping means (e.g. protrusions, not shown) preventing the septum from sliding out of engagement with the mounting means. For illustrative purposes, in fig. 3 the fluid connector is shown in an extended position, however, the connector is arranged to be moved into engagement with the reservoir after this has been mounted, typically just prior to use, this as explained in greater detail below. In the shown embodiment the septum is clamped between a portion of the pump and a separate clamp member, however, one or both of these structures may be formed integrally with housing portions, e.g. an upper and a lower housing portion.

The pump unit further comprises a coil actuator 682 adapted to engage a piston member 683, 340 of a membrane pump (see below). In the shown embodiment the base plate 675 and the clamp member 677 are separate structures, however, these may be formed integrally with e.g. upper respectively lower housing portions.

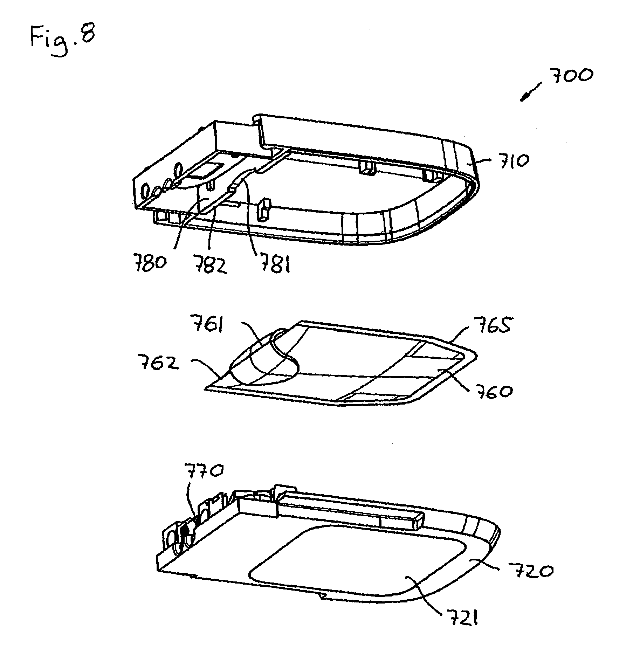

In fig. 8 an exploded view of a further reservoir unit is shown, the unit comprising an upper housing member 710, a lower housing member 720 with a transparent area 721, a flexible reservoir 760 with a rounded edge portion 762 on which a septum member 761 is mounted, a pump assembly 770 with actuator and a circuit board (not shown) arranged above the reservoir and comprising electronic components for controlling actuation of the pump. The upper and lower housing members comprise mounting means in the form of opposed upper and lower ridge portions 780 (the lower not seen) adapted to engage and mount the reservoir in the housing. Each ridge portion comprises a central cut-out portion 781 adapted to engage the septum member on its opposed surfaces when the housing members are assemble thereby locking the reservoir in place within the housing. The degree of locking will be determined by the pressure exerted on the septum member, the elastic properties of the septum member and the friction between the ridge and the septum member. On each side of the cut-out portion the ridge portions comprise a straight portion 782 which may aid in mounting the reservoir in the housing. The straight portions may engage the initially prefilled reservoir to help lock it in place, however, as the reservoir is emptied and flattens this grip may lessen. In contrast, the engagement with the septum is adapted to properly hold the reservoir in place as the reservoir is emptied. The straight portions may also be adapted to pinch and fully flatten the reservoir thus serving as an additional mounting means. Additional mounting means (not shown) may engage and grip the reservoir at other locations, e.g. along the welded edges 765.

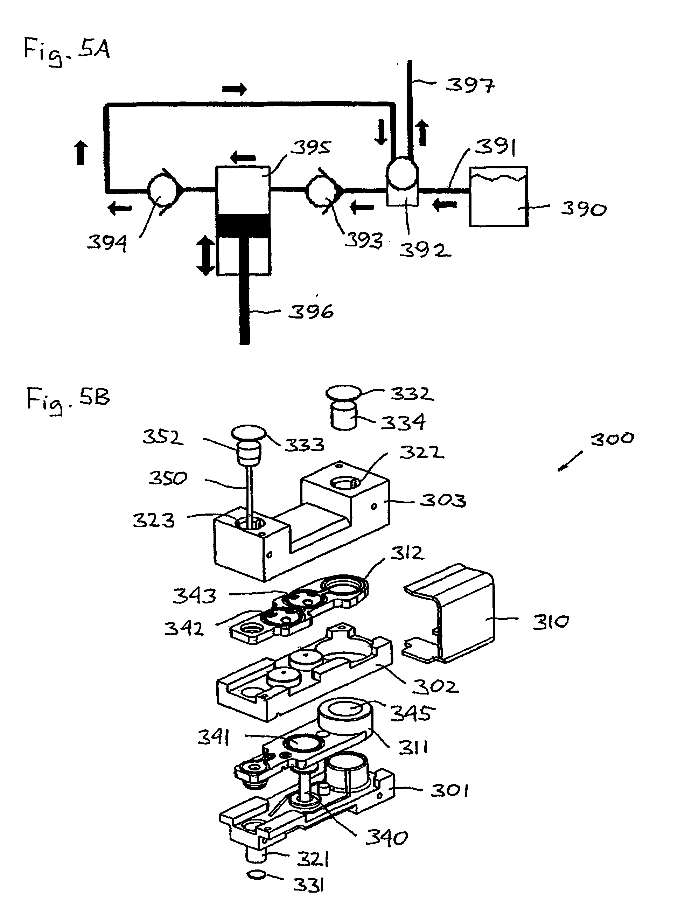

With reference to fig. 5A a schematic overview of a pump connected to a reservoir is shown, the pump comprising the following general features: a fluid connection 391 to reservoir a reservoir 390, a safety valve 392, inlet and outlet valves 393, 394, a pump chamber 395 with an associated piston 396, and an outlet 397. The arrows indicate the flow direction between the individual components. When the piston is moved downwards (in the drawing) a relative negative pressure will build up inside the pump chamber which will cause the inlet valve to open and subsequently fluid will be drawn form the reservoir through the open primary side of the safety valve. When the piston is moved upwards (in the drawing) a relative overpressure will build up in the pump chamber which will cause the inlet valve to close and the outlet valve and the safety valve to open whereby fluid will flow from the pump chamber through the outlet valve and the secondary side of the safety valve to the outlet. As appears, in normal operation the safety valve allows fluid passage during both intake and expelling of fluid and is thus "passive" during normal operation. However, in case the reservoir is pressurized (as may happen for a flexible reservoir) the elevated pressure in the reservoir will be transmitted to both the primary side of the safety valve and, via the pump chamber, the secondary side of the safety valve in which case the pressure on the primary side of the safety valve will prevent the secondary side to open.

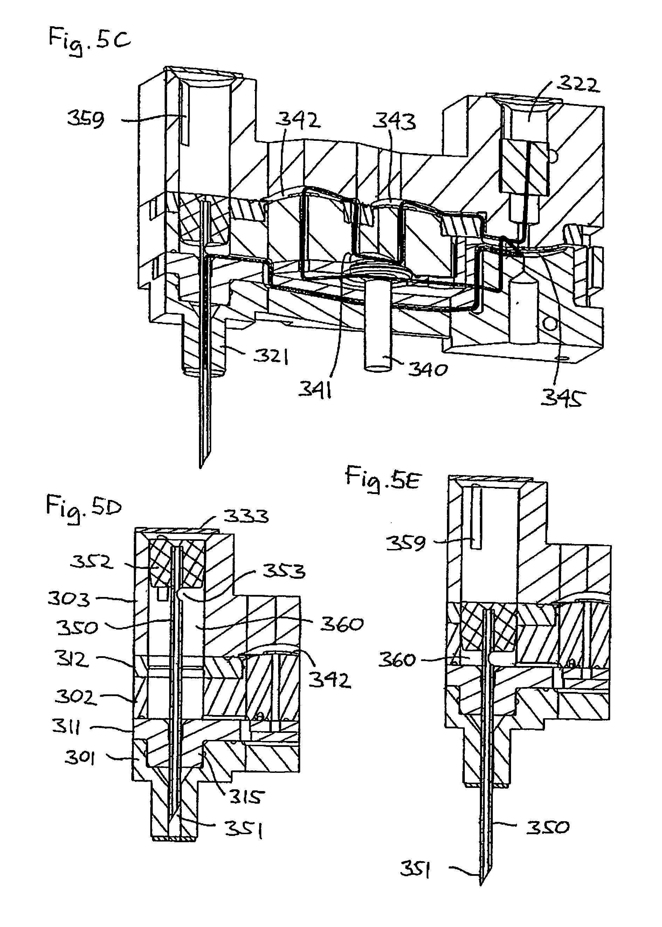

In fig. 5B an exploded view of a pump assembly 300 utilizing the pump principle depicted in fig. 5A is shown, the pump assembly (in the following also referred to as a pump) being suitable for use with the reservoir units of figs. 1-4. The pump is a membrane pump comprising a piston-actuated pump membrane with flow-controlled inlet- and outlet-valves. The pump has a general layered construction comprising first, second and third members 301, 302, 303 between which are interposed first and second membrane layers 311, 312, whereby a pump chamber 341 is formed by the first and second members in combination with the first membrane layer, a safety valve 345 is formed by the first and third members in combination with the first membrane layer, and inlet and outlet valves 342, 343 are formed by the second and third members in combination with the second membrane layer (see fig. 5C). The layers are held in a stacked arrangement by an outer clamp 310. The pump further comprises an inlet 321 and an outlet 322 as well as a connection opening 323 which are all three covered by respective membranes 331, 332, 333 sealing the interior of the pump in an initial sterile state. The membranes are penetratable or breakable (e.g. made from paper) by a needle or other member introduced through a given seal. The outlet further comprises a self-sealing, needle-penetratable septa 334 (e.g. of a rubber-like material) allowing the pump to be connected to an outlet needle. As shown in fig. 5C a fluid path (indicated by the dark line) is formed between the inlet 321 (see below) and the inlet valve 342 via the primary side of the safety valve 345, between the inlet valve, pump chamber 345 and the outlet valve 343, and between the outlet valve and the outlet 322 via the secondary side of the safety valve, the fluid paths being formed in or between the different layers. The pump also comprises a piston 340 for actuating the pump membrane, the piston being driven by external driving means (not shown).

The pump further comprises a fluid connector in the form of hollow connection needle 350 slidably positioned in a needle chamber 360 arranged behind the connection opening, see fig. 5D. The needle chamber is formed through the layers of the pump and comprises an internal sealing septum 315 through which the needle is slidably arranged, the septum being formed by the first membrane layer. The needle comprises a pointed distal end 351, a proximal end on which is arranged a needle piston 352 and a proximal side opening 353 in flow communication with the distal end, the needle and the piston being slidably arranged relative to the internal septum and the chamber. As can be appreciated form fig. 5D the needle piston in its initial position is bypassed by one or more radially placed keyways 359. These are provided in order to allow steam sterilisation and to vent the air otherwise trapped when the fluid connector is moved forward in the needle chamber.

The above-described pump assembly may be provided in a drug delivery device of the type shown in fig. 2. In a situation of use where the reservoir unit is attached to a needle unit a proximal end of the infusion needle is introduced through the outlet seal and septum 334 of the pump, and the connection member 525 (see fig. 2) is introduced through the connection membrane 333. By this action the connection needle is pushed from its initial position as shown in fig. 5D to a actuated position as shown in fig. 5E in which the distal end is moved through the inlet membrane 331 and further through the needle-penetratable septum of a nearby located reservoir, this establishing a flow path between the reservoir and the inlet valve via the proximal opening 353 in the needle. In this position a seal is formed between the needle piston and the needle chamber.

As appears, when the two units are disconnected, the infusion needle 212 is withdrawn from the pump outlet whereas the connection needle permanently provides fluid communication between the pump and the reservoir.

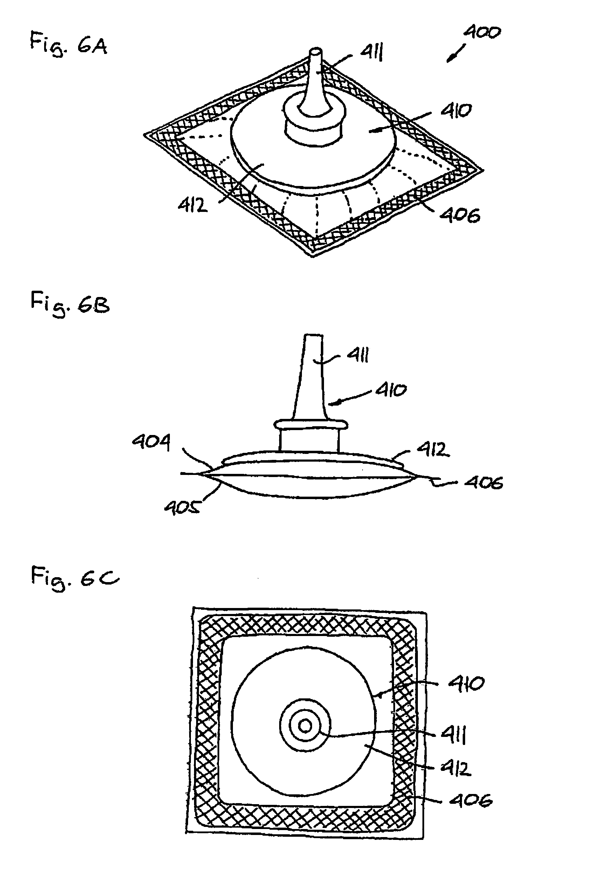

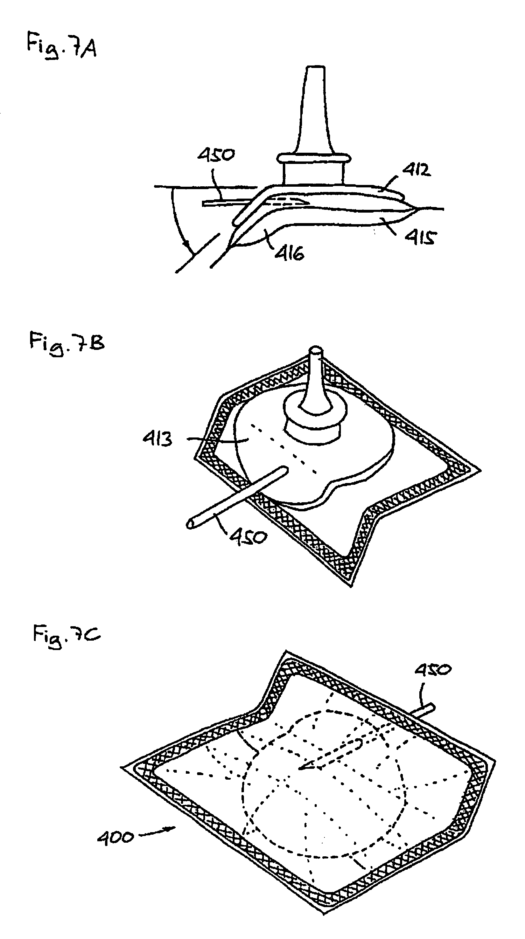

With reference to figs. 6A-6C and 7A-7C an alternative configuration of a flexible, prefilled drug reservoir suitable for use in a delivery device of the type shown in figs. 1 and 2 will be described.

The flexible reservoir 400 comprises first and second flexible wall foil members 404, 405 sealed together at the periphery thereof by welded seams 406, thereby forming a relatively flat pouch for containing the liquid drug, the welded seam of the pouch defining a general plane. An elastomeric septum member 410 is mounted on the first foil member, preferably by welding. The septum member comprises a relatively thin disc-formed base portion from the central portion of which projects an extension 411, the peripheral circumferential part 412 of the base portion forming a needle-penetratable self-sealing connection means. The projection may be utilized e.g. during manufacture and handling of the reservoir, as well as a mounting means for fixating the reservoir relative to other structures without having to interfere with movement of the flexible foil walls.

Although the filled reservoir 400 comprises a generally convex first surface allowing a needle to be inserted therethrough generally in parallel with the general plane of the reservoir, fig. 7A-C shows an arrangement of the reservoir allowing for improved insertion of a needle through the needle-penetratable portion 412 of the septum in parallel with the general plane of the reservoir.

More specifically, by deflecting a portion of the reservoir downwardly relative to the general plane (by e.g. approximately 30 degrees as shown in fig. 7A), an area 413 of the needle-penetratable portion of the septum is "presented" to a needle 450 arranged in parallel with the general plane of the reservoir, thereby allowing for ease of insertion. As shown in fig. 7A, the bend reservoir comprises a first "major" portion 415 being arranged substantially corresponding to the general plane, and a second "minor" portion 416 being deflected relative thereto in a direction away from the first surface, such that a part 413 of the peripheral portion of the septum member is arranged on the second portion of the reservoir. In figs. 7A-7C the reservoir is shown without the means for bending the reservoir, however, such means can be provided by structures of a surrounding housing. The needle 450 may be in the form of a moveable needle connector as described above.

Example: A reservoir containing 3 ml of insulin was manufactured from two foil members of a three-layered laminate comprising an intermediate layer of PCTFE co-extruded with epoxy modified polyethylene imine (a tie-layer) and an inner layer of PE, and with an outer layer of PP laminated on the PCTFE layer. A septum member made from a thermoplastic elastomeric rubber-compound was welded to the outer PP layer before the two foil members were welded to each other along the peripheries thereof, the insulin being filled into the reservoir before it was completely sealed.

In the above description of the preferred embodiments, the different structures and means providing the described functionality for the different components have been described to a degree to which the concept of the present invention will be apparent to the skilled reader. The detailed construction and specification for the different components are considered the object of a normal design procedure performed by the skilled person along the lines set out in the present specification.

## Claims

1. An apparatus (200), comprising:
- a flexible reservoir (260, 400) defining a cavity for containing a fluid, the reservoir having a pouch-like configuration defining a general plane,
- a needle-penetratable outlet (261, 410) arranged on a portion of the reservoir which is inclined relative to the general plane,
- a needle (350, 450) having a generally straight inlet portion and an outlet, the inlet being adapted to be arranged in fluid communication with the reservoir,
- wherein the needle and the reservoir are moveable relative to each from an initial position in which there is no fluid communication there between and a connected position in which the needle inlet is arranged in fluid communication with the reservoir through the reservoir outlet,
- wherein the reservoir has a first surface and an opposed second surface, at least the first surface having, in a fluid filled state of the reservoir, a generally convex configuration relative to the interior of the reservoir and the general plane, the outlet being arranged on the first surface, and
- wherein the needle in the connected position penetrates the reservoir outlet substantially in parallel with the general plane, **characterized in that**
- the reservoir comprises a first major and second minor portion (415, 416), the first portion being arranged substantially corresponding to the general plane, the second portion being deflected relative to the general plane in a direction away from the first surface, the reservoir outlet being arranged on the second portion of the reservoir.

2. An apparatus as in claim 1, the reservoir comprising first and second flexible foil portions (404, 405) sealed together to form the cavity.

3. An apparatus as in claim 2, comprising a septum member (410) formed from a needle-penetratable self-sealing material, the septum member forming the reservoir outlet.

4. An apparatus as in claim 3, wherein the septum member has been mounted on a foil portion of the reservoir by means of welding.

5. An apparatus as in any claim 3, further comprising:
- a housing (251),
- mounting means (562, 675, 677, 780) arranged within or formed by the housing,
- wherein the mounting means engages the septum member to thereby mount the flexible reservoir relative to the housing.

6. An apparatus as in claim 5, wherein substantially the entire flexible reservoir apart from the outlet means is free to move relative to the housing.

7. An apparatus as in any of claims 1-6, further comprising an expelling assembly (300, 670) having an inlet (321, 672) and an outlet (322) and an internal flow path arranged there between, the inlet being adapted to be arranged in fluid communication with the reservoir via the needle, the expelling assembly being adapted to expel a fluid contained in the reservoir through the outlet of the expelling assembly.

8. An apparatus as in claim 7, wherein the expelling assembly comprises a needle connector (350) serving as the inlet for the expelling assembly as well as the needle for connection to the reservoir,
- wherein the needle connector (350) is arranged within the interior of the expelling assembly in an initial state, the fluid connector comprising an inlet (351) and an outlet (353), whereby the needle connector is arranged to be operated from the initial state and to an operating state in which fluid communication is established between the interior of the reservoir and the interior of the expelling assembly via the needle connector and with the outlet of the needle connector being arranged in the flow path of the expelling assembly.

9. An apparatus as in claim 7 or 8, further comprising:
- a transcutaneous device (212) adapted to penetrate the skin of a subject,
- a mounting surface (232) adapted for application to the skin of the subject,
- wherein the reservoir comprises a fluid drug, and
- the expelling assembly, in a situation of use, is adapted for expelling the drug out of the reservoir and through the skin of the subject via the transcutaneous device.

## Patentansprüche

1. Vorrichtung (200), umfassend:
- einen flexiblen Behälter (260, 400), der einen Hohlraum zum Aufnehmen eines Fluids definiert, wobei der Behälter eine beutelartige Konfiguration aufweist, die eine allgemeine Ebene definiert,
- einen nadeldurchdringbaren Auslass (261, 410), der auf einem Bereich des Behälters angeordnet ist, der in Bezug auf die allgemeine Ebene geneigt ist,
- eine Nadel (350, 450) mit einem im Allgemeinen geraden Einlassbereich und einem Auslass, wobei der Einlass derart angepasst ist, dass er mit dem Behälter in Fluidkommunikation gebracht werden kann,
- wobei die Nadel und der Behälter relativ zueinander von einer anfänglichen Position, in welcher zwischen ihnen keine Fluidkommunikation vorliegt, zu einer verbundenen Position, in welcher der Nadeleinlass mit dem Behälter durch den Behälterauslass in Fluidkommunikation angeordnet ist, beweglich sind,
- wobei der Behälter eine erste Oberfläche und eine gegenüberliegende zweite Oberfläche aufweist, wobei zumindest die erste Oberfläche in mit Fluid gefülltem Zustand des Behälters eine im Allgemeinen konvexe Konfiguration in Bezug auf das Innere des Behälters und die allgemeine Ebene aufweist, wobei der Auslass an der ersten Oberfläche angeordnet ist, und
- wobei die Nadel in der verbundenen Position den Behälterauslass im Wesentlichen parallel mit der allgemeinen Ebene durchdringt,
**dadurch gekennzeichnet dass**
- der Behälter einen ersten Haupt- und einen zweiten Nebenbereich (415, 416) umfasst, wobei der erste Bereich im Wesentlichen entsprechend der allgemeinen Ebene angeordnet ist, wobei der zweite Bereich in Bezug auf die allgemeine Ebene in einer Richtung weg von der ersten Oberfläche abgelenkt ist, wobei der Behälterauslass an dem zweiten Bereich des Behälters angeordnet ist.

2. Vorrichtung nach Anspruch 1, wobei der Behälter erste und zweite flexible Folienbereiche (404, 405) umfasst, die unter Bildung des Hohlraums miteinander versiegelt sind.

3. Vorrichtung nach Anspruch 2, umfassend ein Septumelement (410), das aus einem nadeldurchdringbaren selbstabdichtenden Material gebildet ist, wobei das Septumelement den Behälterauslass bildet.

4. Vorrichtung nach Anspruch 3, wobei das Septumelement mittels Verschweißen an einem Folienbereich des Behälters befestigt ist.

5. Vorrichtung nach Anspruch 3, ferner umfassend:
- ein Gehäuse (251),
- Befestigungsmittel (562, 675, 677, 780), die in dem Gehäuse angeordnet oder durch Selbiges geformt sind,
- wobei das Befestigungsmittel in das Septum eingreift, um **dadurch** den flexiblen Behälter in Bezug auf das Gehäuse zu befestigen.

6. Vorrichtung nach Anspruch 5, wobei im Wesentlichen der gesamte flexible Behälter abgesehen vom Auslassmittel für eine Bewegung in Bezug auf das Gehäuse frei ist.

7. Vorrichtung nach einem der Ansprüche 1-6, ferner umfassend eine Ausstoßanordnung (300, 670) mit einem Einlass (321, 672) und einem Auslass (322) und einem dazwischen angeordneten inneren Fließweg, wobei der Einlass derart angepasst ist, dass er mit dem Behälter über die Nadel in Fluidkommunikation angeordnet ist, wobei die Ausstoßanordnung derart angepasst ist, dass sie ein im Behälter enthaltenes Fluid durch den Auslass der Ausstoßanordnung ausstößt.

8. Vorrichtung nach Anspruch 7, wobei die Ausstoßanordnung ein Nadelverbindungsstück (350) umfasst, das als der Einlass für die Ausstoßanordnung sowie als die Nadel zum Verbinden mit dem Behälter dient,
- wobei das Nadelverbindungsstück (350) in einem anfänglichen Zustand im Inneren der Ausstoßanordnung angeordnet ist, wobei das Fluidverbindungsstück einen Einlass (351) und einen Auslass (353) umfasst, wodurch das Nadelverbindungsstück angeordnet ist, um es von dem anfänglichen Zustand und zu einem Betriebszustand, in welchem zwischen dem Inneren des Behälters und dem Inneren der Ausstoßanordnung über das Nadelverbindungsstück Fluidkommunikation gegeben ist, zu betreiben, und wobei der Auslass des Nadelverbindungsstücks im Fließweg der Ausstoßanordnung angeordnet ist.

9. Vorrichtung nach Anspruch 7 oder 8, ferner umfassend:
- eine transkutane Vorrichtung (212), die dazu angepasst ist, die Haut eines Patienten zu durchdringen,
- eine Befestigungsoberfläche (232), die zum Anbringen an die Haut des Patienten angepasst ist,
- wobei der Behälter einen fluidförmigen Arzneistoff umfasst, und
- wobei die Ausstoßanordnung in einer Verwendungssituation derart angepasst ist, dass sie den Arzneistoff aus dem Behälter und durch die Haut des Patienten über die transkutane Vorrichtung ausstößt.

## Revendications

1. Un dispositif (200) comprenant :
- un réservoir flexible (260, 400) définissant une cavité pour contenir un fluide, le réservoir ayant une configuration semblable à une poche définissant un plan d'ensemble,
- une sortie pénétrable par aiguille (261, 410) disposée sur une partie du réservoir qui est inclinée par rapport au plan d'ensemble,
- une aiguille (350, 450) ayant une partie d'entrée généralement rectiligne et une sortie, l'entrée étant apte à être disposée en communication de fluide avec le réservoir,
- dans lequel l'aiguille et le réservoir sont mobiles l'un par rapport à l'autre depuis une position initiale où il n'y a pas communication de fluide entre eux et une position connectée où l'entrée de l'aiguille est disposée en communication de fluide avec le réservoir au travers de la sortie du réservoir,
- dans lequel le réservoir présente une première surface et une seconde surface opposée, au moins la première surface ayant, dans un état rempli de fluide du réservoir, une configuration généralement convexe par rapport à l'intérieur du réservoir et au plan d'ensemble, la sortie étant disposée sur la première surface, et
- dans lequel l'aiguille dans la position connectée pénètre la sortie du réservoir substantiellement en parallèle avec le plan d'ensemble, **caractérisé en ce que**
- le réservoir comprend une première partie majeure et une seconde partie mineure (415, 416), la première partie étant disposée substantiellement en correspondance avec le plan d'ensemble, la seconde partie étant déviée par rapport au plan d'ensemble dans une direction en éloignement de la première surface, la sortie du réservoir étant disposée sur la seconde partie du réservoir.

2. Un dispositif selon la revendication 1, avec le réservoir comprenant une première et une seconde parties de feuille flexible (404, 405) scellées ensemble pour former la cavité.

3. Un dispositif selon la revendication 2, comprenant un organe de septum (410) formé à partir d'un matériau auto-obturant pénétrable par aiguille, l'organe de septum formant la sortie du réservoir.

4. Un dispositif selon la revendication 3, dans lequel l'organe de septum a été monté sur une partie de feuille de réservoir au moyen d'un soudage.

5. Un dispositif selon la revendication 3, comprenant en outre :
- un boîtier (251),
- des moyens de montage (562, 675, 677, 780) disposés à l'intérieur du boîtier ou formés par le boîtier,
- dans lequel les moyens de montage viennent en contact avec l'organe de septum afin de monter de cette manière le réservoir flexible par rapport au boîtier.

6. Un dispositif selon la revendication 5, dans lequel substantiellement la totalité du réservoir flexible à l'exception des moyens de sortie est libre de se déplacer par rapport au boîtier.

7. Un dispositif selon l'une des revendications 1 à 6, comprenant en outre un bloc d'expulsion (300, 670) ayant une entrée (321, 672) et une sortie (322) et un trajet d'écoulement interne disposé entre celles-ci, l'entrée étant apte à être disposée en communication de fluide avec le réservoir via l'aiguille, le bloc d'expulsion étant apte à expulser un fluide contenu dans le réservoir au travers de la sortie du bloc d'expulsion.

8. Un dispositif selon la revendication 7, dans lequel le bloc d'expulsion comprend un connecteur d'aiguille (350) servant d'entrée pour le bloc d'expulsion en même temps que l'aiguille pour connexion au réservoir,
- dans lequel le connecteur d'aiguille (350) est disposé à l'intérieur du bloc d'expulsion dans un état initial, le connecteur de fluide comprenant une entrée (351) et une sortie (353), de sorte que le connecteur d'aiguille soit disposé pour être actionné à partir de l'état initial et jusqu'à un état opérant dans lequel la communication de fluide est établie entre l'intérieur du réservoir et l'intérieur du bloc d'expulsion via le connecteur d'aiguille et avec la sortie du connecteur d'aiguille disposée dans le trajet d'écoulement du bloc d'expulsion.

9. Un dispositif selon la revendication 7 ou 8, comprenant en outre :
- un dispositif transcutané (212) apte à pénétrer la peau d'un patient,
- une surface de montage (232) apte à une application sur la peau du patient,
- dans lequel le réservoir comprend un médicament fluide, et
- le bloc d'expulsion, dans une situation d'utilisation, est apte à expulser le médicament hors du réservoir et au travers de la peau du patient via le dispositif transcutané.
